# FASCICULE DE BREVET EUROPEEN

(11) **EP 1 993 484 B1**
(45) Date de publication et mention de la délivrance du brevet: **17.07.2019**
(21) Numéro de dépôt: 07731130.6
(22) Date de dépôt: 13.03.2007
(51) Int. Cl.: A61F 2/44

(54) **PROTHESES DE DISQUES INTERVERTEBRALES**
BANDSCHEIBENPROTHESEN
INTERVERTEBRAL DISC PROSTHESES

(30) Priorité: 14.03.2006 FR 0602226
(43) Date de publication de la demande: 26.11.2008
(73) Titulaire: Spineart SA, 1217 Meyrin (CH)
(72) Inventeur: LEVIEUX, Jérôme, CH-1293 Bellevue (CH)
(74) Mandataire: Cabinet Plasseraud
(86) Numéro de dépôt international: PCT/FR2007/000434
(87) Numéro de publication internationale: WO 2007/104860

(56) Documents cités:
- WO-A2-2004/016217
- FR-A1- 2 775 587
- FR-A1- 2 787 014
- FR-A1- 2 799 116
- US-A1- 2004 143 334
- US-A1- 2004 243 238
- US-A1- 2005 165 485

## Description

La présente invention concerne des prothèses de disques intervertébrales.

Des dégénérescences dues à un traumatisme, des maladies ou la vieillesse conduisent fréquemment au remplacement d'un disque intervertébral.

Ces dégénérescences peuvent entraîner une altération de l'espace naturel entre deux vertèbres. Le rétrécissement de cet espace naturel peut entraîner une pression qui s'exerce sur certains nerfs, et par conséquent des douleurs peuvent apparaître.

Une prothèse de disque peut être utilisée pour maintenir l'espace naturel entre deux vertèbres.

Elle doit aussi permettre aux vertèbres de se déplacer l'une par rapport à l'autre selon un mouvement naturel. Notamment elle doit permettre un mouvement de rotation axial qui correspond en partie au mouvement de rotation du tronc ou du cou en cervical du corps humain, un mouvement antéropostérieur qui correspond à un mouvement de flexion ou d'extension de la partie supérieure du corps ou de la tête en cervical, et un mouvement latéral qui correspond à une inclinaison de la partie supérieure du corps, ou du cou en cervical.

Les plateaux supérieur et inférieur d'une prothèse de disque sont en appui sur les vertèbres et avec le temps s'y fixent grâce aux traitements de surface et aux aspérités dont ils sont généralement pourvus.

EP-A1-1 344 508 décrit une prothèse de disque intervertébral comportant un plateau supérieur, un plateau inférieur présentant une surface d'appui, un élément intermédiaire comportant une surface inférieure et une surface supérieure présentant une partie sphérique, le plateau supérieur présentant une empreinte complémentaire de la partie sphérique et venant au contact de la partie sphérique de manière à définir une liaison rotule entre le plateau supérieur et l'élément intermédiaire, la surface inférieure de l'élément intermédiaire venant au contact de la surface d'appui.

L'élément intermédiaire peut se déplacer d'avant en arrière sur la surface d'appui du plateau inférieur.

Cependant ce type de prothèses n'offre pas le confort et la robustesse souhaités, notamment lors de chocs violents.

WO2004/016247 décrit des prothèses de disques intervertébrales. Une grande variété de configurations est proposée. Dans l'une d'elles, sont prévus un plateau inférieur, un plateau supérieur et un élément intermédiaire comprenant une surface supérieure convexe pour coopérer avec une concavité ménagée dans le plateau supérieur. L'élément intermédiaire comprend de longues et minces entailles horizontales.

Cependant ce type de prothèses, pour produire un amortissement suffisant, nécessiterait des entailles très profondes, créant des amorces de rupture en fatigue qui fragiliseraient considérablement la prothèse et n'offrirait notamment pas la robustesse souhaitée, particulièrement lors de chocs violents ou en rotation.

La présente invention a notamment pour but de pallier ces inconvénients.

C'est pourquoi la présente demande a pour objet une prothèse de disque intervertébral comportant
- un plateau supérieur ayant une surface externe d'appui sur une vertèbre supérieure et une surface dirigée vers l'intérieur de la prothèse,
- un plateau inférieur ayant une surface externe d'appui sur une vertèbre inférieure et une surface dirigée vers l'intérieur de la prothèse et
- une liaison rotule entre le plateau supérieur et le plateau inférieur, comprenant un élément bombé convexe, de préférence sphérique, coopérant avec une empreinte complémentaire de l'élément bombé convexe et venant au contact dudit élément bombé convexe, ledit élément bombé convexe comprenant au moins une lumière lui permettant de se déformer lors des mouvements brusques ou vibratoires de la vie courante d'un individu, en particulier en cas de choc vertical.

La déformation de l'élément bombé lors des mouvements brusques ou vibratoires de la vie courante (choc, saut, accident, chute, effort violent, sport contraignant (ski, cheval...)) d'un individu consistera essentiellement en un écrasement dudit élément bombé convexe, c'est-à-dire une diminution de sa hauteur. De préférence, aucune déformation de l'élément bombé n'aura lieu lors des mouvements normaux de la vie courante tels que la marche à pieds.

Dans des conditions de mise en oeuvre de l'invention, une prothèse de disque intervertébral ci-dessus est réalisée en trois éléments.

L'élément de la prothèse comportant l'élément bombé convexe est alors une pièce intermédiaire installée entre les deux plateaux.

Par exemple, l'ensemble peut comprendre un plateau inférieur présentant une surface externe d'appui sur une vertèbre inférieure et une surface dirigée vers l'intérieur de la prothèse servant de surface d'appui pour un élément intermédiaire comportant une surface inférieure et une surface supérieure présentant une partie bombée, de préférence sphérique, et un plateau supérieur ayant une surface externe d'appui sur une vertèbre supérieure et une surface dirigée vers l'intérieur de la prothèse présentant une empreinte complémentaire de la partie bombée et venant au contact de ladite partie bombée de manière à définir une liaison rotule entre le plateau supérieur et l'élément intermédiaire.

L'élément intermédiaire peut aussi correspondre au seul élément bombé convexe, qui sera alors installé dans un chambrage prévu dans le plateau inférieur. Il aura alors en général une forme de cylindre court surmonté d'une calotte bombée, notamment sphérique.

L'évidement prévu dans l'élément bombé convexe comprend au moins une lumière. Celle-ci peut avoir toute forme, de préférence régulière et comportant au moins un axe de symétrie, par exemple cubique ou parallélépipédique, sphérique, oblongue etc. Rappelons que dans le cadre de la présente invention, une lumière est une cavité fermée, ce qui la distingue d'une entaille qui est ouverte sur l'extérieur.

On peut trouver une ou plusieurs lumières parfois ci-après appelées aussi évidements. Celles-ci seront de préférence installées régulièrement par rapport aux axes de symétrie de la partie bombée. Cependant, une installation asymétrique sera préférée, par exemple pour effectuer une correction ou conférer un effet particulier.

La ou les lumières peuvent avoir une forme de fentes horizontale(s), alternées ou empilées ou obliques dans le cas de pluralité de fentes, une forme de trous jointifs ou espacés, de forme notamment parallélépipédique ou de préférence circulaire, alignés ou non, ou une forme de trous reliés par une fente longitudinale, vus en coupe transversale.

La nature du matériau utilisé pour la réalisation de l'élément présentant une partie bombée conditionne la structure et la taille du ou des évidements réalisés.

La forme et le nombre d'évidements seront choisis en fonction de l'amortissement souhaité. A matériau utilisé identique, plus les dimensions de l'évidement seront importantes, plus l'élément bombé convexe sera déformable. De même, plus le nombre et ou le volume des évidements sera important, plus ledit élément bombé sera déformable.

L'homme de l'art peut avec quelques expériences simples déterminer la forme, la taille des lumières et le matériau utilisé pour atteindre la déformabilité désirée.

L'évidement est réalisé à l'intérieur de la partie bombée sans déboucher à l'extérieur de celle-ci.

Le ou les évidement(s) pourront présenter une forme convexe, par exemple en forme de calotte, ou encore une forme sensiblement sphérique ou oblongue.

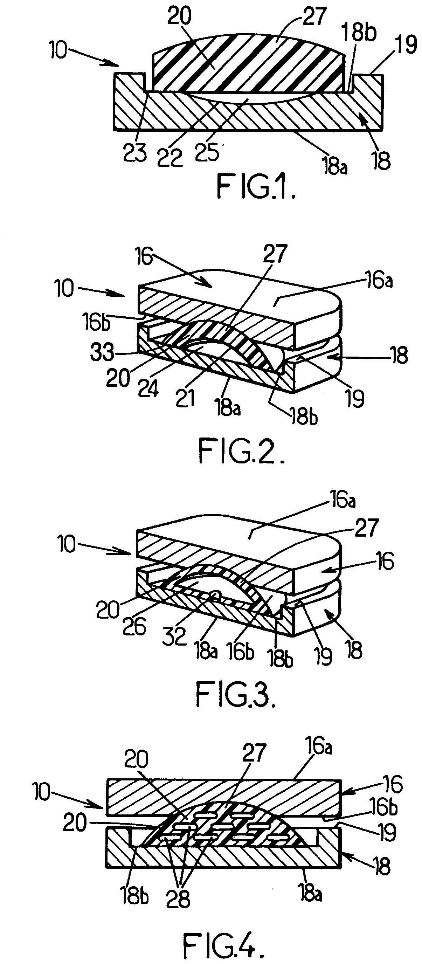

On a, dans une prothèse de disque intervertébral ci-dessus réalisée en trois éléments, une prothèse dont :
- la surface inférieure de l'élément intermédiaire présente une partie concave débouchant sur l'extérieur,
- la surface inférieure de l'élément intermédiaire présente une partie concave débouchant sur l'extérieur et comporte une partie plane autour de la partie concave et le rapport de surface entre la partie concave et la partie plane est par exemple compris entre 1/5 et 9/10, avantageusement compris entre 1/4 et 9/10, notamment compris entre 1/4 et 4/5, particulièrement compris entre 1/4 et 3/4, tout particulièrement compris entre 1/3 et ¾.
- la surface d'appui présente une partie de forme concave, dans ce cas la lumière est constituée par cette concavité, fermée par l'élément intermédiaire comme représenté ci-après à la figure 1.
- la surface d'appui comporte une partie plane autour de la partie concave et le rapport de surface entre la partie concave et la partie plane est par exemple compris entre 1/5 et 9/10, avantageusement compris entre 1/4 et 9/10, notamment compris entre 1/4 et 4/5, particulièrement compris entre 1/4 et 3/4, tout particulièrement compris entre 1/3 et 3/4,
- le plateau inférieur comporte des rebords délimitant la surface d'appui.

La cavité constituée par la partie concave pouvant être de très faible épaisseur (quelques dixièmes de millimètres), une réalisation ne présentant pas d'anneau plan autour de la partie concave fonctionnera de la même manière ; en effet, cet anneau se formera naturellement dès la mise en charge. Dans ce cas la surface de l'anneau ne sera pas tout à fait plane. C'est le cas par exemple avec un évidement de forme sphérique à grand rayon de courbure.

L'homme de l'art comprendra facilement que si par exemple la surface inférieure de l'élément intermédiaire présente une partie concave débouchant sur l'extérieur et prend appui sur une surface plane du plateau inférieur dirigée vers l'intérieur de la prothèse, un même effet d'amortissement peut être obtenu (fig2). De même, en prévoyant une concavité dans la surface du plateau inférieur dirigée vers l'intérieur de la prothèse, tandis que l'élément intermédiaire ne comprend aucun évidement, un même effet d'amortissement peut être obtenu (fig1). De telles variantes de réalisation équivalentes entrent dans la portée de l'invention. Il comprendra facilement aussi que les adjectifs "inférieur" et "supérieur" ont un sens relatif, car une prothèse de l'invention peut être installée dans un sens ou l'autre. Ainsi, dans le cas d'une prothèse de disque intervertébral ci-dessus réalisée en deux éléments, le plateau "inférieur" présentant une partie bombée pourra se trouver du côté des pieds ou au contraire de la tête de l'individu chez qui elle est installée.

Une partie bombée sphérique aura par exemple un diamètre externe de 0,5 à 10 cm, de préférence de 1 à 8 cm, notamment de 1,5 à 7 cm, tout particulièrement de 2 à 5 cm, dans le cas d'une prothèse lombaire, que la réalisation soit en 2 ou 3 éléments (ou plus).

Une partie bombée sphérique aura par exemple un diamètre externe de 0,3 à 8 cm, de préférence de 0,5 à 7 cm, notamment de 0,8 à 5 cm, tout particulièrement de 1 à 3 cm, dans le cas d'une prothèse cervicale, que la réalisation soit en 2 ou 3 éléments (ou plus).

Les différents éléments pourront être réalisés en des matériaux différents ou en un même matériau. De préférence l'élément comprenant un élément bombé convexe, notamment l'élément intermédiaire dans une prothèse de disque intervertébral ci-dessus comprenant trois éléments, est réalisé en résine thermoplastique ou en pyrocarbone tel que celui commercialisé par la société BioProfile (Grenoble France) sous la dénomination Pyc ®. La résine thermoplastique est par exemple du polyéthylène à haut poids moléculaire et de préférence du polyétheréthercétone (PEEK), chargé en fibres de verre ou carbone mais de préférence vierge.

Dans le cas d'une prothèse de disque intervertébral ci-dessus réalisée en trois éléments,
- le plateau supérieur peut être réalisé dans un matériau du type chrome - cobalt ou titane ou acier inoxydable. Le plateau supérieur peut aussi être réalisé en polyétheréthercétone (PEEK) possédant de préférence un revêtement de surface en titane sur sa face supérieure. Le plateau supérieur peut aussi être réalisé en matériau du type chrome - cobalt ou titane ou acier inoxydable et posséder un traitement de surface durcissant (notamment de type Diamolith® commercialisé par la société IonBond ou Innovative Coatings Company - Le Mée sur Seine FRANCE) ou un insert en pyrocarbone afin d'améliorer ses propriétés de frottement et d'usure.
- l'élément intermédiaire peut être réalisé dans un matériau plastique, de type polyéthylène haute densité, ayant de très bonnes caractéristiques de glissement,
- l'élément intermédiaire peut aussi être réalisé en matériau tel que du polyétheréthercétone (PEEK) ou en pyrocarbone qui présente un module de Young d'environ 24 Mpa qui lui confère une certaine élasticité lors des chocs.
- le plateau inférieur peut être réalisé dans un matériau du type chrome - cobalt ou titane ou acier inoxydable. Le plateau inférieur peut aussi être réalisé en polyétheréthercétone (PEEK) possédant un revêtement de surface en titane sur sa face inférieure. Le plateau supérieur peut aussi être réalisé en matériau du type chrome - cobalt ou titane ou acier inoxydable et posséder un traitement de surface durcissant (notamment de type Diamolith® commercialisé par la société IonBond) ou un insert en pyrocarbone afin d'améliorer ses propriétés de frottement et d'usure.

Dans des conditions préférentielles de mise en oeuvre de l'invention, une prothèse de disque intervertébral ci-dessus est réalisée en trois éléments et les plateaux inférieur et supérieur possèdent sur leur face interne un revêtement de surface durcissant. Dans d'autres conditions préférentielles de mise en oeuvre de l'invention, les plateaux inférieur et supérieur possèdent sur leur face interne un insert en pyrocarbone.

Les prothèses objet de la présente invention possèdent de très intéressantes propriétés et qualités.

Le ou les évidement(s) prévu(s) dans l'élément bombé convexe permet(tent) par déformation de l'élément bombé un amortissement des chocs auxquels la prothèse est soumise.

Elles présentent ainsi un confort d'utilisation et une robustesse améliorée par rapport aux prothèses de l'art antérieur. Notamment elles permettent d'amortir les chocs par déformation de la matière de l'élément bombé, et permettent ainsi d'éviter le descellement de la prothèse.

Ces propriétés sont illustrées ci-après dans la partie expérimentale. Elles justifient l'utilisation des prothèses ci-dessus décrites, dans le remplacement d'un disque intervertébral sujet à des dégénérescences suite à un traumatisme, des maladies ou la vieillesse.

A cette fin, elles peuvent être installées chez un individu de telle sorte que la convexité soit dirigée vers le bas ou de préférence vers le haut.

C'est pourquoi le présent exposé décrit également une méthode de remplacement d'un disque intervertébral, sujet par exemple à des dégénérescences suite à un traumatisme, des maladies ou la vieillesse, dans laquelle on implante entre deux vertèbres adjacentes au moins une prothèse ci-dessus.

Avantageusement, le plateau présentant une empreinte complémentaire de la partie bombée est installé au dessus de celui comprenant la partie bombée.

Les conditions préférentielles de mise en oeuvre des prothèses intervertébrales ci-dessus décrites s'appliquent également aux autres objets de l'invention visés ci-dessus, notamment aux méthodes de remplacement d'un disque intervertébral.

L'invention sera mieux comprise si l'on se réfère aux dessins annexés sur lesquels,
- la figure 1 représente une vue schématique de côté d'une partie d'une prothèse de disque selon un premier mode de réalisation en trois éléments (deux représentés),
- les figures 2 et 3 représentent une vue en perspective coupée selon un plan vertical d'une prothèse de disque selon un deuxième et un troisième mode de réalisation en trois éléments,
- la figure 4 représente une vue en coupe de côté d'une prothèse de disque selon un quatrième mode de réalisation en trois éléments,
- la figure 5 représente une vue de côté d'une prothèse de disque en trois éléments disposée entre deux vertèbres,
- les figures 6, 7 et 8 représentent une vue en coupe de côté d'une prothèse de disque selon un mode de réalisation en deux éléments qui ne correspond pas à l'invention.

Sur les différentes figures, les mêmes références désignent des éléments identiques ou similaires.

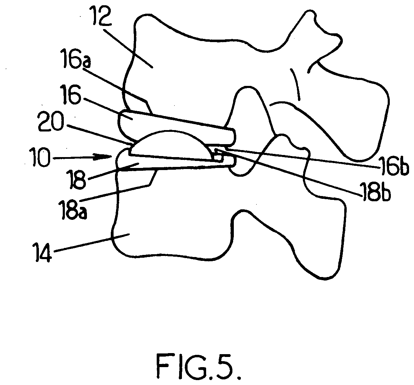

La prothèse de disque 10 est destinée à être disposée entre deux vertèbres d'une colonne vertébrale 12, 14. Par exemple la figure 5 illustre une vue de profil de deux vertèbres 12, 14 d'une colonne vertébrale entre lesquelles est disposée une prothèse 10 selon l'invention.

La prothèse 10 de disque comporte un plateau supérieur 16 et un plateau inférieur 18 présentant chacun une face externe 16a, 18a orientée vers respectivement une vertèbre supérieure et un vertèbre inférieure. Sur chacune des faces externes 16a, 18a sont disposées des reliefs par exemple en dents de scie, non représentés, qui permettent l'ancrage des plateaux 16, 18 dans les vertèbres.

Les plateaux supérieur 16 et inférieur 18 comprennent aussi chacun une surface interne 16b, 18b. Les surfaces internes s'étendent latéralement selon un premier axe antéropostérieur et s'étendent longitudinalement selon un deuxième axe perpendiculaire au premier axe, en vis à vis et elles sont en contact avec un élément intermédiaire 20.

L'élément intermédiaire 20 comporte une surface supérieure présentant un élément bombé convexe 27, sphérique. Il comporte en outre une face inférieure en appui sur la surface interne 18b (qui sera nommée par la suite « surface d'appui 18b ») du plateau inférieur 18. La partie sphérique 27de la face supérieure est en contact avec la surface interne 16b du plateau supérieur 16. La partie bombée 27 permet d'obtenir une liaison de type « rotule » pour autoriser le mouvement dans toutes les directions de l'élément intermédiaire 20 par rapport au plateau supérieur 16, ce qui offre une plus grande flexibilité.

L'élément intermédiaire 20 est monté mobile sur la surface d'appui 18b du plateau inférieur 18. De plus, le plateau inférieur comporte un rebord 19 délimitant la surface d'appui 18b.

La liaison rotule entre le plateau supérieur 16 fixé avec le temps à la vertèbre supérieure 12 et l'élément intermédiaire 20 relié lui-même à la vertèbre inférieure 14 par le plateau inférieur 18 permet de reproduire les mouvements de flexion ou d'extension et les mouvements d'inclinaison entre deux vertèbres 12, 14 d'une colonne vertébrale.

Tel que représenté à la figure 1, le plateau inférieur 18 comporte, sur sa face d'appui 18b, une partie 22 de forme concave et une partie plane 23, l'ensemble faisant face à la partie inférieure de l'élément intermédiaire 20, de manière à former un évidement 25. Cette partie concave 22 présente une dimension de surface inférieure à celle de la surface inférieure de l'élément intermédiaire 20, de telle manière que l'élément intermédiaire 20 puisse se déplacer sur la partie plane 23 de la surface d'appui 18b du plateau inférieur 18. Le rapport de surface entre la partie concave et la surface inférieure de l'élément intermédiaire ici représenté est d'environ 3/4. La partie concave 22 crée par ailleurs dans ce mode de réalisation la lumière (25) selon l'invention.

Dans une variante, la cavité constituée par la partie concave est de très faible épaisseur (quelques dixièmes de millimètres). Le plateau inférieur 18 ne comporte pas de partie plane 23. Un anneau correspondant à la partie plane 23 ci-dessus se formera naturellement sur le pourtour du plateau inférieur dès la mise en charge. Dans ce cas la surface de l'anneau ne sera pas tout à fait plane. C'est le cas par exemple avec un évidement de forme sphérique à grand rayon de courbure (de 5 ou de 25 cm de rayon par exemple selon qu'il s'agit d'un dispositif cervical ou lombaire).

Lorsque la prothèse de disque est soumise à des chocs, orientés sensiblement selon une direction verticale, tel que des chutes sur les fesses ou lors d'un saut, une force tend à rapprocher le plateau supérieur 16 du plateau inférieur 18, et l'élément intermédiaire 20 est en conséquence compressé entre les deux plateaux. L'élément intermédiaire 20 se déforme sous l'action de la force exercée par la colonne lors du choc et tend à combler l'espace vide de l'évidement 25, le choc est alors amorti par la déformation de l'élément intermédiaire 20. Les risques de fractures vertébrales ou de descellement sont alors diminués.

Selon d'autres modes de réalisation représentés aux figures 2 à 4, l'évidement a été réalisé sur l'élément intermédiaire.

Sur la figure 2, l'évidement 24 est réalisé sur la face inférieure de l'élément intermédiaire 20. Pour cela la surface inférieure de l'élément intermédiaire présente une partie 21 de forme concave qui fait face à la surface d'appui plane 18b. Cette lumière concave est bouchée par le plateau inférieur 18. Ainsi lorsqu'une force est appliquée sur la prothèse, l'élément intermédiaire 20 se déforme et tend à occuper l'espace formé entre la partie concave 21 et la surface d'appui 18b. La surface inférieure de l'élément intermédiaire comporte en outre une partie plane 33 autour de la partie concave 21, et le rapport de surface entre la partie concave 21 et la partie plane 33 ici représenté est d'environ 1,5. La partie plane est en contact avec la surface d'appui 18b du plateau inférieur 18 et assure ainsi le déplacement par glissement de l'élément intermédiaire 20 sur la surface d'appui 18b. L'évidement débouche à l'extérieur de la partie bombée, ce qui permet l'expulsion du contenu de l'évidement 24 lorsque l'élément intermédiaire 20 est déformé malgré le bouchage par le plateau inférieur 18.

Sur la figure 3, l'évidement 26 est réalisé comme une inclusion dans la partie sphérique 27 de l'élément intermédiaire 20 et forme une cavité. L'élément intermédiaire 20 comporte en outre un passage 32 faisant communiquer la cavité 26 avec l'extérieur de l'élément intermédiaire 20. Ce passage 32 bouché par le plateau inférieur 18 permet cependant l'expulsion du contenu de la cavité lorsque l'élément intermédiaire 20 est déformé, et par conséquent permet un meilleur amortissement de chocs en augmentant les capacités de déformations de l'élément intermédiaire et en évitant les phénomènes de rebonds lors du retour de l'élément intermédiaire à sa forme initiale. Dans cette variante aussi, l'évidement débouche à l'extérieur de la partie bombée.

Sur la figure 4, la partie bombée 27 de l'élément intermédiaire 20 comporte plusieurs lumières ou évidements 28 de forme oblongue en coupe et disposés sensiblement parallèlement à une direction perpendiculaire à l'axe vertical Z, ce qui permet de déformer plus facilement l'élément intermédiaire 20 sous l'action d'une force verticale. Ces évidements ne sont pas reliés à un passage d'expulsion d'air comme décrit précédemment et ne débouchent pas à l'extérieur de celle-ci.

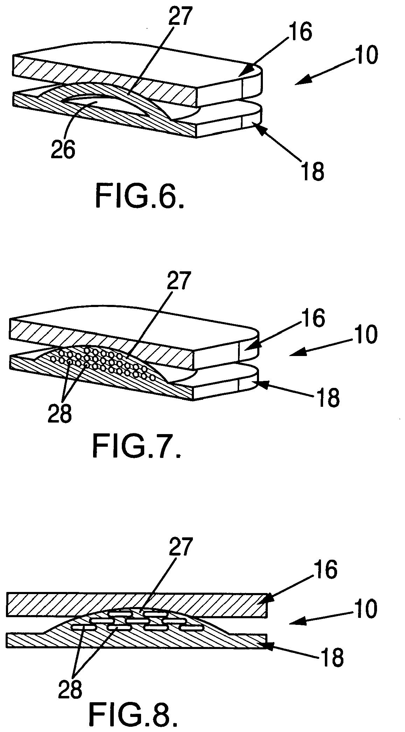

Sur les figures 6, 7, 8 on peut observer une prothèse 10 de disque qui ne correspond pas à l'invention et qui comporte un plateau supérieur 16 et un plateau inférieur 18, ce dernier comportant d'une pièce un élément bombé convexe 27, sphérique, coopérant avec une empreinte complémentaire ménagée dans le plateau supérieur 16.

## Revendications

1. Prothèse de disque intervertébral comportant :
- un plateau supérieur (16) ayant une surface externe d'appui (16a) sur une vertèbre supérieure et une surface (16b) dirigée vers l'intérieur de la prothèse,
- un plateau inférieur (18) ayant une surface externe d'appui (18a) sur une vertèbre inférieure et une surface (18b) dirigée vers l'intérieur de la prothèse ainsi qu'
- un élément intermédiaire (20) formant une liaison rotule entre le plateau supérieur et le plateau inférieur, comprenant un élément bombé convexe (27), coopérant avec une empreinte complémentaire de l'élément bombé convexe (27) et venant au contact dudit élément bombé convexe (27), ledit élément intermédiaire (20) comprenant au moins une lumière (24,25) lui permettant de se déformer lors des mouvements brusques ou vibratoires de la vie courante d'un individu, tels qu'en cas de choc vertical,
**caractérisée en ce que** soit l'élément intermédiaire (20) comprend une surface inférieure présentant une concavité débouchant sur l'extérieur pour prendre appui sur une surface plane du plateau inférieur (18) dirigée vers l'intérieur de la prothèse et ainsi constituer une lumière (24), soit une concavité est prévue dans la surface du plateau inférieur (18) dirigée vers l'intérieur de la prothèse, ladite concavité (25) étant fermée par l'élément intermédiaire (20) pour constituer une lumière (25),
et **caractérisée en ce que** ladite prothèse est réalisée en trois éléments.

2. Prothèse selon la revendication 1, **caractérisée en ce que** la lumière (25) est formée par une concavité prévue dans le plateau inférieur (18).

3. Prothèse selon l'une des revendications 1 et 2 **caractérisée en ce que** l'élément intermédiaire (20) comprend une surface inférieure présentant une concavité débouchant sur l'extérieur pour prendre appui sur une surface plane (18b) du plateau inférieur (18) dirigée vers l'intérieur de la prothèse et ainsi constituer une lumière (24).

4. Prothèse de disque selon l'une des revendications 1 à 3, **caractérisée en ce que** le plateau inférieur (18) comporte des rebords (19) délimitant la surface d'appui de l'élément intermédiaire (20).

5. Prothèse de disque selon l'une des revendications 1 et 3, **caractérisée en ce que** la surface inférieure de l'élément intermédiaire (20) présente une partie concave (24) débouchant sur l'extérieur et comporte une partie plane (33) autour de la partie concave et le rapport de surface entre la partie concave et la partie plane est compris entre 1/5 et 9/10.

6. Prothèse de disque selon l'une des revendications 1 et 2, **caractérisée en ce que** , le plateau inférieur (18) comporte, sur sa face d'appui 18b, une concavité (22) et une partie plane (23), l'ensemble faisant face à la partie inférieure de l'élément intermédiaire (20), de manière à former un évidement (25), cette partie concave (22) présentant une dimension de surface inférieure à celle de la surface inférieure de l'élément intermédiaire (20), de telle manière que l'élément intermédiaire (20) puisse se déplacer sur la partie plane (23) de la surface d'appui (18b) du plateau inférieur (18).

7. Prothèse de disque selon l'une des revendications 1 à 6, **caractérisée en ce que** l'élément intermédiaire (20) est réalisé en polyétheréthercétone.

8. Prothèse selon l'une des revendications 1 à 7, **caractérisée en ce que** les plateaux inférieur et supérieur possèdent sur leur face interne (16b) et (18b) un revêtement de surface durcissant.

9. Prothèse selon l'une des revendications 1 à 7, **caractérisée en ce que** les plateaux inférieur et supérieur possèdent sur leur face interne (16b) et (18b) un insert en pyrocarbone.

10. Prothèse selon l'une des revendications 1 à 9, **caractérisée en ce que** les éléments de la prothèse sont réalisés en polyétheréthercétone.

## Patentansprüche

1. Bandscheibenprothese, umfassend:
- eine obere Platte (16), welche eine externe Fläche für eine Anlage (16a) an einem oberen Wirbel und eine Fläche (16b) aufweist, welche in Richtung des Inneren der Prothese gerichtet ist,
- eine untere Platte (18), welche eine externe Fläche für eine Anlage (18a) an einem unteren Wirbel und eine Fläche (18b) aufweist, welche in Richtung des Inneren der Prothese gerichtet ist, sowie
- ein Zwischenelement (20), welches eine Kugelverbindung zwischen der oberen Platte und der unteren Platte bildet, umfassend ein gewölbtes konvexes Element (27), welches mit einer Vertiefung zusammenwirkt, welche komplementär zu dem gewölbten konvexen Element (27) ist und mit dem gewölbten konvexen Element (27) in Kontakt kommt, wobei das Zwischenelement (20) wenigstens ein Lumen (24, 25) umfasst, welches ihm erlaubt, sich während plötzlicher Bewegungen oder Vibrationen des täglichen Lebens eines Individuums zu verformen, wie beispielsweise im Fall eines vertikalen Stoßes,
**dadurch gekennzeichnet, dass** das Zwischenelement (20) eine untere Fläche umfasst, welche eine Konkavität aufweist, welche an der Außenseite mündet, um eine Anlage an einer ebenen Fläche der unteren Platte (18) einzunehmen, welche in Richtung des Inneren der Prothese gerichtet ist, und somit ein Lumen (24) zu bilden, d.h., dass eine Konkavität in der Fläche der unteren Platte (18) vorgesehen ist, welche in Richtung des Inneren der Prothese gerichtet ist, wobei die Konkavität (25) durch das Zwischenelement (20) geschlossen ist, um ein Lumen (25) zu bilden,
und **dadurch gekennzeichnet, dass** die Prothese aus drei Elementen gebildet ist.

2. Prothese nach Anspruch 1, **dadurch gekennzeichnet, dass** das Lumen (25) durch eine Konkavität gebildet ist, welche in der unteren Platte (18) vorgesehen ist.

3. Prothese nach einem der Ansprüche 1 und 2, **dadurch gekennzeichnet, dass** das Zwischenelement (20) eine untere Fläche umfasst, welche eine Konkavität aufweist, welche an der Außenseite mündet, um eine Anlage an einer ebenen Fläche (18b) der unteren Platte (18) einzunehmen, welche in Richtung des Inneren der Prothese gerichtet ist, und somit ein Lumen (24) zu bilden.

4. Scheibenprothese nach einem der Ansprüche 1 bis 3, **dadurch gekennzeichnet, dass** die untere Platte (18) Ränder (19) umfasst, welche die Anlagefläche des Zwischenelements (20) begrenzen.

5. Scheibenprothese nach einem der Ansprüche 1 und 3, **dadurch gekennzeichnet, dass** die untere Fläche des Zwischenelements (20) einen konkaven Abschnitt (24) aufweist, welcher an der Außenseite mündet und einen ebenen Abschnitt (33) um den konkaven Abschnitt herum umfasst, und das Flächenverhältnis zwischen dem konkaven Abschnitt und dem ebenen Abschnitt zwischen 1/5 und 9/10 beträgt.

6. Scheibenprothese nach einem der Ansprüche 1 und 2, **dadurch gekennzeichnet, dass** die untere Platte (18) an ihrer Anlagefläche (18b) eine Konkavität (22) und einen ebenen Abschnitt (23) umfasst, wobei die Einheit zu dem unteren Abschnitt des Zwischenelements (20) derart weist, dass eine Aussparung (25) gebildet ist, wobei der konkave Abschnitt (22) eine Flächenabmessung aufweist, welche kleiner als diejenige der unteren Fläche des Zwischenelements (20) ist, so dass sich das Zwischenelement (20) an dem ebenen Abschnitt (23) der Anlagefläche (18b) der unteren Platte (18) verlagern kann.

7. Scheibenprothese nach einem der Ansprüche 1 bis 6, **dadurch gekennzeichnet, dass** das Zwischenelement (20) aus Polyetheretherketon hergestellt ist.

8. Prothese nach einem der Ansprüche 1 bis 7, **dadurch gekennzeichnet, dass** die unteren und oberen Platten an ihrer inneren Fläche (16b) und (18b) eine härtende Oberflächenauflage besitzen.

9. Prothese nach einem der Ansprüche 1 bis 7, **dadurch gekennzeichnet, dass** die unteren und oberen Platten an ihrer inneren Fläche (16b) und (18b) einen Einsatz aus Pyrokarbon besitzen.

10. Prothese nach einem der Ansprüche 1 bis 9, **dadurch gekennzeichnet, dass** die Elemente der Prothese aus Polyetheretherketon hergestellt sind.

## Claims

1. Intervertebral disc prosthesis comprising:
- an upper plate (16) having an outer surface (16a) for bearing on an upper vertebra and a surface (16b) oriented towards the inside of the prosthesis,
- a lower plate (18) having an outer surface (18a) for bearing on a lower vertebra and a surface (18b) oriented towards the inside of the prosthesis as well as
- an intermediate element (20) forming a ball joint between the upper plate and the lower plate, comprising a convex bulging element (27), cooperating with an imprint complementary to the convex bulging element (27) and in contact with said convex bulging element (27), said intermediate element (20) comprising at least one hole (24,25) allowing it to deform during the sudden or vibrational movements of the daily life of an individual, such as in the case of a vertical impact,
**characterised in that** either the intermediate element (20) comprises a lower surface having a concavity opening towards the outside in order to bear on a flat surface of the lower plate (18) oriented towards the inside of the prosthesis and thus form a hole (24), or a concavity is provided in the surface of the lower plate (18) oriented towards the inside of the prosthesis, said concavity (25) being closed by the intermediate element (20) in order to form a hole (25),
and **characterised in that** said prosthesis is made from three elements.

2. Prosthesis according to claim 1, **characterised in that** the hole (25) is formed by a concavity provided in the lower plate (18).

3. Prosthesis according to one of claims 1 and 2, **characterised in that** the intermediate element (20) comprises a lower surface having a concavity opening towards the outside in order to bear on a flat surface (18b) of the lower plate (18) oriented towards the inside of the prosthesis and thus form a hole (24).

4. Disc prosthesis according to one of claims 1 to 3, **characterised in that** the lower plate (18) comprises rims (19) defining the bearing surface of the intermediate element (20).

5. Disc prosthesis according to one of claims 1 and 3, **characterised in that** the lower surface of the intermediate element (20) has a concave portion (24) opening towards the outside and comprises a flat portion (33) around the concave portion and the surface-area ratio between the concave portion and the flat portion is between 1/5 and 9/10.

6. Disc prosthesis according to one of claims 1 and 2, **characterised in that**, the lower plate (18) comprises, on its bearing face (18b), a concavity (22) and a flat portion (23), the assembly facing the lower portion of the intermediate element (20), in such a way as to form a recess (25), this concave portion (22) having a surface dimension smaller than that of the lower surface of the intermediate element (20), in such a way that the intermediate element (20) can move on the flat portion (23) of the bearing surface (18b) of the lower plate (18).

7. Disc prosthesis according to one of claims 1 to 6, **characterised in that** the intermediate element (20) is made from polyether ether ketone.

8. Prosthesis according to one of claims 1 to 7, **characterised in that** the lower and upper plate have, on their inner face (16b) and (18b), a hardening surface coating.

9. Prosthesis according to one of claims 1 to 7, **characterised in that** the lower and upper plate have, on their inner face (16b) and (18b), an insert made of pyrocarbon.

10. Prosthesis according to one of claims 1 to 9, **characterised in that** the elements of the prosthesis are made from polyether ether ketone.
